(19) Europäisches Patentamt
European Patent Office
Office européen des brevets

(11) Veröffentlichungsnummer: **0 123 218**
**A2**

# (12) EUROPÄISCHE PATENTANMELDUNG

(21) Anmeldenummer: 84104095.9

(22) Anmeldetag: 12.04.84

(51) Int. Cl.³: **C 07 D 249/08**
C 07 D 233/60, A 01 N 43/64
A 01 N 43/50

(30) Priorität: 23.04.83 DE 3314738

(43) Veröffentlichungstag der Anmeldung:
31.10.84 Patentblatt 84/44

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Stetter, Jörg, Dr.
Gellertweg 4
D-5600 Wuppertal 1(DE)

(72) Erfinder: Büchel, Karl Heinz, Prof. Dr.
Dabringhausener Strasse 42
D-5093 Burscheid(DE)

(72) Erfinder: Reinecke, Paul, Dr.
Lessingstrasse 11
D-5090 Leverkusen 3(DE)

(72) Erfinder: Brandes, Wilhelm, Dr.
Eichendorffstrasse 3
D-5653 Leichlingen 1(DE)

**(54) Azolylethyl-benzyl-ether-Derivate, Verfahren zu ihrer Herstellung und ihre Verwendung als Fungizide.**

(57) Die Anmeldung betrifft neue Azolylethyl-benzyl-ether-Derivate, ein Verfahren zu ihrer Herstellung sowie ihre Verwendung als Fungizide.

Die neuen Verbindungen der Formel

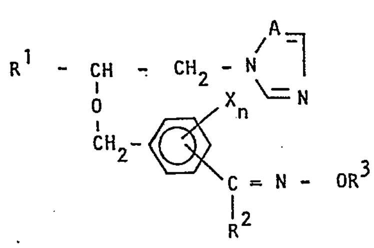

(I)

in welcher

A, R¹, R², R³, X und n die in der Beschreibung angegebene Bedeutung besitzen, werden erhalten, wenn man Azolylethanole mit Benzylhalogeniden umsetzt. Die Produkte weisen eine mikrobizide Wirkung auf. Sie sind für den Gebrauch als Pflanzenschutzmittel ungeeignet und können mit besonders gutem Erfolg zur Bekämpfung von Getreidekrankheiten, von Venturia Arten und von Reiskrankheiten eingesetzt werden.

EP 0 123 218 A2

BAYER AKTIENGESELLSCHAFT 5090 Leverkusen, Bayerwerk

Konzernverwaltung RP
Patentabteilung

Slr/Ke
Ia

## Azolylethyl-benzyl-ether-Derivate, Verfahren zu ihrer Herstellung und ihre Verwendung als Fungizide

Die vorliegende Anmeldung betrifft neue Azolylethyl-benzyl-ether-Derivate, ein Verfahren zu ihrer Herstellung sowie ihre Verwendung als Fungizide.

Es ist bereits bekannt geworden, daß bestimmte Azolylethyl-ether-Derivate, wie beispielsweise 1-Butyloxy-1-(2,4-dichlorphenyl)-2-(1,2,4-triazol-1-yl)-ethan oder 1-(4-Chlorbenzyloxy)-1-(2,4-dichlorphenyl)-2-(imidazol-1-yl)-ethan, allgemein gute fungizide Eigenschaften besitzen(vergleiche DE-OS 25 47 953 [LeA 16 749] und US-PS 3 717 655). Die Wirkung dieser Verbindungen ist jedoch in bestimmten Indikationsbereichen, insbesondere bei niedrigen Aufwandmengen und -konzentrationen, nicht immer ganz befriedigend.

Es wurden neue Azolylethyl-benzyl-ether-Derivate der allgemeinen Formel

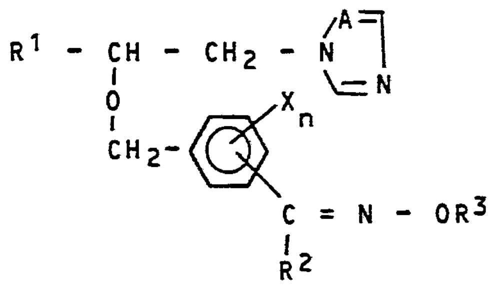

(I)

in welcher

A für ein Stickstoffatom oder die CH-Gruppe steht,

$R^1$ für gegebenenfalls substituiertes Phenyl steht,

$R^2$ für Wasserstoff, Alkyl oder gegebenenfalls substituiertes Phenyl steht,

$R^3$ für Wasserstoff, Alkyl, Alkenyl oder Alkinyl steht,

X für Halogen, Alkyl, Alkoxy, Alkylthio, Halogenalkyl, Halogenalkoxy oder Halogenalkylthio steht und

n für die Zahlen 0, 1 oder 2 steht,

sowie deren Säureadditions-Salze und Metallsalz-Komplexe gefunden.

Die Verbindungen der Formel (I) können in der syn- und anti-Form vorliegen; vorwiegend fallen sie als Gemische beider Formen an.

Weiterhin wurde gefunden, daß man die Azolylethyl-benzyl-ether-Derivate der Formel (I) erhält, wenn man Azolylethanole der Formel

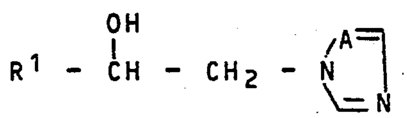

(II)

in welcher

A und $R^1$ die oben angegebene Bedeutung haben,

mit Benzylhalogeniden der Formel

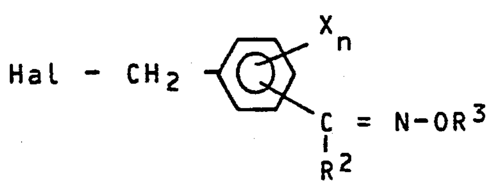

(III)

in welcher

Hal für Halogen, insbesondere Brom oder Chlor, steht und

$R^2$, $R^3$, X und n die oben angegebene Bedeutung haben,

in Gegenwart einer Base und in Gegenwart eines organischen Verdünnungsmittels, oder in einem wässrig-organischen Zweiphasensystem in Gegenwart eines Phasentransferkatalysators, umsetzt.

An die so erhaltenen Verbindungen der Formel (I) können gegebenenfalls anschließend eine Säure oder ein Metallsalz addiert werden.

Die neuen Azolylethyl-benzyl-ether-Derivate weisen starke fungizide Eigenschaften auf. Dabei zeigen überraschenderweise die erfindungsgemäßen Verbindungen eine höhere Wirkung als die aus dem Stand der Technik bekannten Azolylethyl-ether-Derivate 1-Butyloxy-1-(2,4-dichlorphenyl)-2-(1,2,4-triazol-1-yl)-ethan und 1-(4-Chlorbenzyloxy)-1-(2,4-dichlorphenyl)-2-(imidazol-1-yl)-ethan, welche konstitutionell und wirkungsmäßig naheliegende Verbindungen sind. Die erfindungsgemäßen Stoffe stellen somit eine Bereicherung der Technik dar.

Die erfindungsgemäßen Azolylethyl-benzyl-ether-Derivate sind durch die Formel (I) allgemein definiert. In dieser Formel stehen vorzugsweise

A für ein Stickstoffatom oder die CH-Gruppe;

$R^1$ für gegebenenfalls einfach oder zweifach, gleich oder verschieden substituiertes Phenyl, wobei als Substituenten vorzugsweise genannt seien: Halogen, Alkyl mit 1 bis 4 Kohlenstoffatomen, Alkoxy mit 1 bis 2 Kohlenstoffatomen, Halogenalkyl und Halogenalkoxy mit jeweils 1 bis 2 Kohlenstoffatomen und 1 bis 5 gleichen oder verschiedenen Halogenatomen, wie Fluor- und Chloratomen sowie gegebenenfalls durch Halogen substituiertes Phenyl und Phenoxy;

$R^2$ für Wasserstoff, geradkettiges oder verzweigtes Alkyl mit 1 bis 4 Kohlenstoffatomen oder für gegebenenfalls einfach oder zweifach, gleich oder verschieden substituiertes Phenyl, wobei als Substituenten vorzugsweise die bei $R^1$ bereits genannten Phenylsubstituenten infrage kommen;

$R^3$ für Wasserstoff, geradkettiges oder verzweigtes Alkyl mit 1 bis 4 Kohlenstoffatomen oder für Alkenyl und Alkinyl mit jeweils 2 bis 4 Kohlenstoffatomen;

X für Halogen, geradkettiges oder verzweigtes Alkyl, Alkoxy und Alkylthio mit jeweils 1 bis 4 Kohlenstoffatomen oder Halogenalkyl, Halogenalkoxy und Halogenalkylthio mit jeweils 1 bis 2 Kohlenstoffatomen und 1 bis 5 gleichen oder verschiedenen Halogenatomen, wie Fluor- und Chloratomen; und der Index

n für die Zahlen 0, 1 oder 2.

Besonders bevorzugt sind diejenigen Verbindungen der Formel (I), in denen

A für ein Stickstoffatom oder die CH-Gruppe steht;

$R^1$ für gegebenenfalls einfach oder zweifach, gleich oder verschieden substituiertes Phenyl steht, wobei als Substituenten genannt seien: Fluor, Chlor, Brom, Methyl, Ethyl, Methoxy, Trifluormethyl, Trifluormethoxy, Phenyl, Chlorphenyl und Phenoxy;

$R^2$ für Wasserstoff, Methyl, Ethyl, Isopropyl, oder für gegebenenfalls einfach oder zweifach, gleich oder verschieden durch Fluor, Chlor, Methyl, Methoxy und Trifluormethyl substituiertes Phenyl steht;

$R^3$ für Wasserstoff, Methyl, Ethyl, n-Propyl, n-Butyl, Allyl oder Propargyl steht;

X für Fluor, Chlor, Brom, Methyl, Methoxy, Methylthio, Trifluormethyl, Trifluormethoxy oder Trifluormethylthio steht; und der Index

n für die Zahlen 0 oder 1 steht.

Ganz besonders bevorzugt sind diejenigen Verbindungen der Formel (I), in denen

A für ein Stickstoffatom oder die CH-Gruppe steht;

$R^1$ für gegebenenfalls einfach oder zweifach, gleich oder verschieden durch Chlor oder Methyl substituiertes Phenyl steht;

$R^2$ für Wasserstoff oder Methyl steht;

$R^3$ für Methyl steht; und der Index

n den Wert 0 annimmt.

Bevorzugte erfindungsgemäße Verbindungen sind auch Additionsprodukte aus Säuren und denjenigen Azolylethyl-benzylether-Derivaten der Formel (I), in denen die Substituenten A, $R^1$, $R^2$, $R^3$ und X sowie der Index n die Bedeutungen haben, die bereits vorzugsweise für diese Substituenten und den Index n genannt wurden.

Zu den Säuren, die addiert werden können, gehören vorzugsweise Halogenwasserstoffsäuren, wie z.B. Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure und Milchsäure, sowie Sulfonsäuren, wie p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Azolylethyl-benzyl-ether-Derivaten der Formel (I), in denen die Substituenten A, $R^1$, $R^2$, $R^3$ und X sowie der Index n die Bedeutungen haben, die bereits vorzugsweise für diese Substituenten und den Index n genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Verwendet man beispielsweise 1-(2,4-Dichlorphenyl)-2-(1,2,4-triazol-1-yl)-2-ethanol und 4-Methoxyiminomethyl-benzylbromid als Ausgangsstoffe, so kann der Reaktionsablauf des erfindungsgemäßen Verfahrens durch das folgende Formelschema wiedergegeben werden:

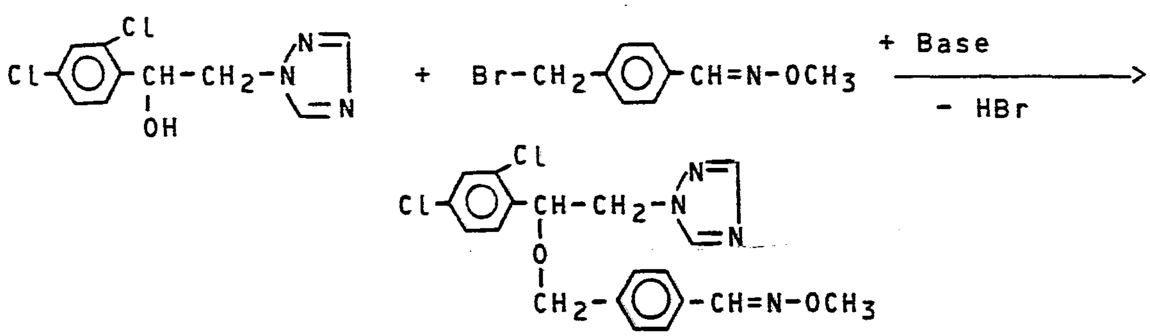


Die bei der Durchführung des erfindungsgemäßen Verfahrens als Ausgangsstoffe benötigten Azolylethanole sind durch die Formel (II) allgemein definiert. In dieser Formel stehen A und $R^1$ vorzugsweise für die Bedeutungen, die bei der Beschreibung der erfindungsgemäßen Stoffe der Formel (I) bereits vorzugsweise für diese Reste genannt wurden.

Die Azolylethanole der Formel (II) sind bekannt (vergleiche z.B. DE-OS 24 31 407, 26 38 470 und 19 40 388); bzw. können sie in allgemein bekannter Art und Weise erhalten werden, indem man die entsprechenden α-Brom(Chlor)ketone mit Imidazol oder 1,2,4-Triazol in Gegenwart eines inerten organischen Lösungsmittels, wie beispielsweise Aceton, und in Gegenwart eines Säurebindemittels, wie beispielsweise Kaliumcarbonat, vorzugsweise in der Siedehitze umsetzt und die so erhaltenen Azolylethanone in üblicher Weise mit komplexen Hydriden, wie beispielsweise Natriumhydrid, oder mit Aluminiumisopropylat reduziert.

Die außerdem für das erfindungsgemäße Verfahren als Ausgangsstoffe benötigten Benzylhalogenide sind durch die Formel(III) allgemein definiert. In dieser Formel stehen $R^2$, $R^3$, X und der Index n vorzugsweise für die Bedeutungen, die bei der Beschreibung der erfindungsgemäßen Stoffe der Formel (I) bereits vorzugsweise für diese Reste und den Index genannt wurden.

Die Benzylhalogenide der Formel (III) sind bekannt, bzw. können sie in allgemein bekannter Art und Weise erhalten werden, indem man entsprechende Carbonylverbindungen mit Hydroxylamin-(Derivaten) in Gegenwart eines Lösungsmittels, vorzugsweise eines Alkohols, bei Temperaturen zwischen 20 und 100°C umsetzt. Dabei wird das Hydroxylamin-(Derivat) vorzugsweise in Form eines Salzes, insbesondere als Hydrochlorid, gegebenenfalls in Gegenwart eines Säurebindemittels, wie beispielsweise Natriumacetat, eingesetzt. Die Isolierung erfolgt in üblicher Art und Weise.

Die Benzylbromide der Formel (III) können auch erhalten werden, indem man entsprechende Toluol-Derivate in allgemein bekannter Art und Weise mit N-Bromsuccinimid umsetzt.

Als Verdünnungsmittel kommen für das erfindungsgemäße Verfahren vorzugsweise inerte organische Lösungsmittel infrage. Hierzu gehören vorzugsweise Ketone, wie Diethylketon, insbesondere Aceton und Methylethylketon; Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Als Basen können für das erfindungsgemäße Verfahren alle üblichen organischen und anorganischen Basen eingesetzt werden. Hierzu gehören vorzugsweise tertiäre Amine, beispielsweise Triethylamin, oder Pyridin und Alkalihydroxide oder Alkalicarbonate, beispielsweise Natrium- und Kaliumhydroxid, sowie Alkalimetallhydride wie beispielsweise Natriumhydrid.

Die Reaktionstemperaturen können bei der Durchführung des erfindungsgemäßen Verfahrens in einem größeren Bereich variiert werden. Im allgemeinen arbeitet man zwischen 0°C und 150°C, vorzugsweise zwischen 20°C und 100°C.

Bei der Durchführung des erfindungsgemäßen Verfahrens arbeitet man vorzugsweise mit äquimolaren Mengen. Es ist jedoch auch möglich, eine der Komponenten in einem Ueberschuß einzusetzten. Die Aufarbeitung und Isolierung der Reaktionsprodukte erfolgt nach üblichen Methoden.

In einer bevorzugten Ausführungsform wird die erfindungsgemäße Umsetzung in einem Zweiphasensystem, wie beispielsweise wässige Natron-oder Kalilauge/Toluol oder Methylenchlorid durchgeführt, gegebenenfalls unter Zusatz von 0,1 - 1 Mol eines Phasentransfer-Katalysators, wie beispielsweise einer Ammonium- oder Phosphoniumverbindung, beispielsweise seien Benzyl-dodecyl-dimethyl-ammoniumchlorid und Triethyl-benzyl-ammoniumchlorid genannt.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren , isoliert und gegebenenfalls durch Waschen mit einem inerten organischen Lösungsmittel gereinigt werden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie Leptosphaeria nodorum, Mehltau, Rost, Cochliobolus sativus und Pyrenophora teres; ferner von Venturia Arten, wie Venturia inaequalis und von Reiskrankheiten, wie Pyricularia oryzae und Pellicularia sasakii, eingesetzt werden.
Die erfindungsgemäßen Stoffe zeigen außerdem gute fungizide in-vitro Eigenschaften.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.
Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

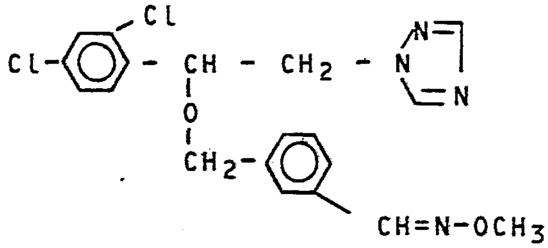

12,9g (0,05 Mol) 1-(2,4-Dichlorphenyl)-2-(1,2,4-triazol-1-yl)-ethanol und 0,5g Triethylbenzylammoniumchlorid werden in einem 2-Phasengemisch aus 100 ml Methylenchlorid und 15 ml 50%iger Natronlauge gelöst und unter schnellem Rühren tropfenweise mit 12,9g (0,06 Mol) 3-Brommethyl-benzaldehyd-O-methyloximether versetzt. Das Gemisch wird 3 Stunden bei Raumtemperatur nachgerührt, die wässrige Phase abgetrennt und die organische Phase mehrmals mit Wasser gewaschen.
Nach Trocknen über Natriumsulfat wird die organische Phase eingeengt und der Rückstand mit Diisopropylether verrührt. Man erhält 9,2g (45 % der Theorie) 1-(2,4-Dichlorphenyl)-1-(3-methoximinomethyl-benzyloxy)-2-(1,2,4-triazol-1-yl)-ethan vom Schmelzpunkt 125- 128°C.

Herstellung des Ausgangsproduktes

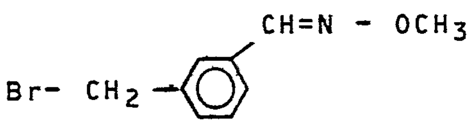

Ein Gemisch aus 44,7g (0,3 Mol) 3-Methylbenzaldehyd-O-methyloximether, 53,7g (0,3 Mol) N-Bromsuccinimid und 0,5g Azodiisobutyronitril in 200 ml Tetrachlorkohlenstoff wird 4 Stunden unter Rückfluß erhitzt. Nach Abfiltrieren des entstandenen Succinimids wird die Lösung am Rotationsverdampfer eingeengt und der Rückstand destilliert. Man erhält 37,6g (55 % der Theorie) 3-Brommethyl-benzaldehyd-O-methyloximether vom Siedepunkt 90-95°C/0,5 mbar.

In entsprechender Weise und gemäß dem erfindungsgemäßen Verfahren werden die Verbindungen der folgenden Tabelle der allgemeinen Formel

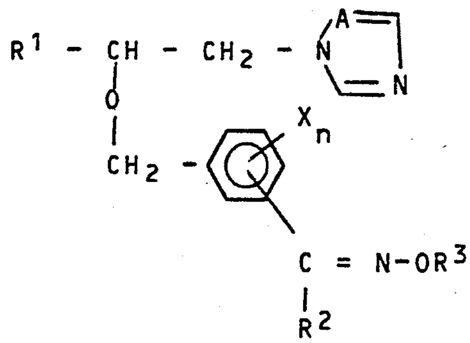

(I)

erhalten:

| Bsp. | $R^1$ | 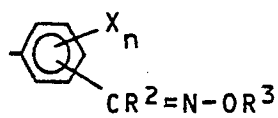 | A | Physikal. Konstante |
|---|---|---|---|---|
| 2 | 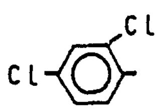 | 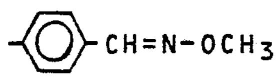 | N | zähes Oel |
| 3 | 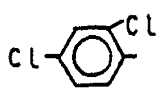 | 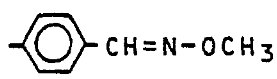 | CH | zähes Oel |
| 4 | 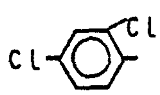 | 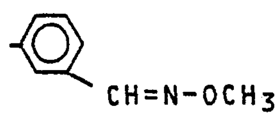 | CH | zähes Oel |

NMR-Daten für obige Beispiele ($CDCl_3$ in TMS):

Beispiel 2:

s 3,9 ppm (3H); m 4,3 ppm (2H); s 4,35 ppm (2H); m 5,1 ppm (1H); g 7,2 ppm (4H); m 7,35 ppm (3H); s 7,85 ppm (1H); s 7,95 ppm (1H); s 8,05 ppm (1H);

Beispiel 3:

s 3,95 ppm (3H); m 4,1 ppm (2H); m 4,35 ppm (2H); m 4,95 ppm (1H); m 7,2 ppm (10H); s 8,0 ppm (1H);

Beispiel 4:

s 3,95 ppm (3H); m 4,1 ppm (2H); m 4,3 ppm (2H);
m 4,9 ppm (1H); m 6,85 ppm (2H); m 7,25 ppm (8H);
s 7,95 ppm (1H)

Verwendungsbeispiele

In den nachfolgenden Beispielen werden die nachstehend angegebenen Verbindungen als Vergleichssubstanzen eingesetzt:

(A) 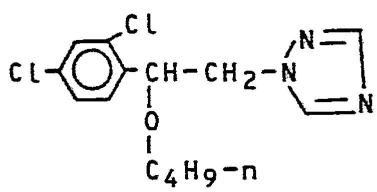

(B) 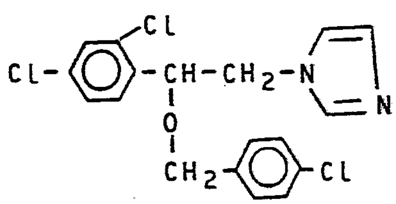 x $HNO_3$

Beispiel A

Leptosphaeria nodorum-Test (Weizen) / protektiv

Lösungsmittel: 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.
Die Pflanzen werden im Gewächshaus bei einer Temperatur von ca. 15°C und einer relativen Luftfeuchtigkeit von ca. 80% aufgestellt.

10 Tage nach der Inokulation erfolgt die Auswertung.

Eine deutliche Ueberlegenheit in der Wirksamkeit gegenüber dem Stand der Technik zeigen bei diesem Test z.B. die Verbindungen gemäß folgender Herstellungsbeispiele: 3, 1 und 4.

Beispiel B

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 2, 3 und 1.

Patentansprüche

1. Azolylethyl-benzyl-ether-Derivate der allgemeinen Formel

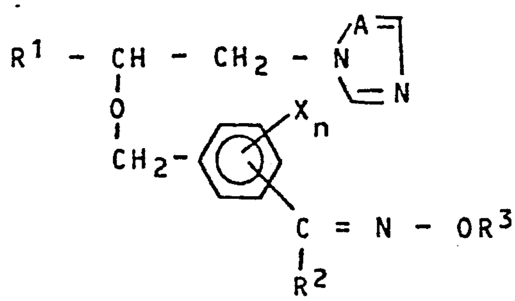

(I)

in welcher

A für ein Stickstoffatom oder die CH-Gruppe steht,

$R^1$ für gegebenenfalls substituiertes Phenyl steht,

$R^2$ für Wasserstoff, Alkyl oder gegebenenfalls substituiertes Phenyl steht,

$R^3$ für Wasserstoff, Alkyl, Alkenyl oder Alkinyl steht,

X für Halogen, Alkyl, Alkoxy, Alkylthio, Halogenalkyl, Halogenalkoxy oder Halogenalkylthio steht und

n für die Zahlen 0, 1 oder 2 steht,

sowie deren Säureadditions-Salze und Metallsalz-Komplexe.

2. Verbindungen der Formel (I) in Anspruch 1, wobei

A für ein Stickstoffatom oder die CH-Gruppe steht,

$R^1$ für Phenyl steht, welches substituiert sein kann durch Halogen, Alkyl mit 1 bis 4 Kohlenstoffatomen, Alkoxy mit 1 bis 2 Kohlenstoffatomen, Halogenalkyl und Halogenalkoxy mit jeweils 1 bis 2 Kohlenstoffatomen und 1 bis 5 gleichen oder verschiedenen Halogenatomen, sowie durch gegebenenfalls selbst durch Halogen substituiertes Phenyl und Phenoxy,

$R^2$ für Wasserstoff, für geradkettiges oder verzweigtes Alkyl mit 1 bis 4 Kohlenstoffatomen steht, oder für gegebenenfalls substituiertes Phenyl steht, wobei als Substituenten die bei $R^1$ bereits genannten Phenylsubstituenten infrage kommen,

$R^3$ für Wasserstoff, für geradkettiges oder verzweigtes Alkyl mit 1 bis 4 Kohlenstoffatomen oder für Alkenyl und Alkinyl mit jeweils 2 bis 4 Kohlenstoffatomen steht,

X für Halogen, für geradkettiges oder verzweigtes Alkyl, für Alkoxy und Alkylthio mit jeweils 1 bis 4 Kohlenstoffatomen oder für Halogenalkyl, Halogenalkoxy und Halogenalkylthio mit jeweils 1 bis 2 Kohlenstoffatomen und 1 bis 5 gleichen oder verschiedenen Halogenatomen steht, und

n für die Zahlen 0, 1 oder 2 steht.

3. Verbindungen der Formel (I) in Anspruch 1, wobei

A für ein Stickstoffatom oder die CH-Gruppe steht,

$R^1$ für gegebenenfalls einfach oder zweifach, gleich oder verschieden durch Fluor, Chlor, Brom, Methyl, Ethyl, Methoxy, Trifluormethyl, Trifluormethoxy, Phenyl, Chlorphenyl und Phenoxy substituiertes Phenyl steht,

$R^2$ für Wasserstoff, Methyl, Ethyl, Isopropyl, oder für gegebenenfalls einfach oder zweifach, gleich oder verschieden durch Fluor, Chlor, Methyl, Methoxy und Trifluormethyl substituiertes Phenyl steht,

$R^3$ für Wasserstoff, Methyl, Ethyl, n-Propyl, n-Butyl, Allyl oder Propargyl steht,

X für Fluor, Chlor, Brom, Methyl, Methoxy, Methylthio, Trifluormethyl, Trifluormethoxy oder Trifluormethylthio steht, und der Index

n für die Zahlen 0 oder 1 steht.

4. Verfahren zur Herstellung von Azolylethyl-benzylether-Derivaten der allgemeinen Formel

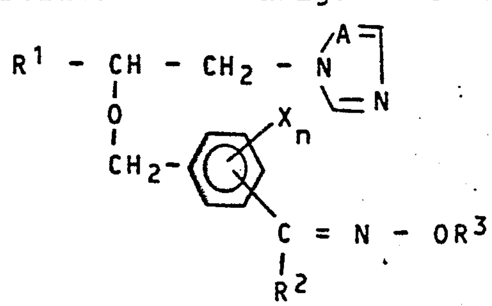

(I)

in welcher

A für ein Stickstoffatom oder die CH-Gruppe steht,

$R^1$ für gegebenenfalls substituiertes Phenyl steht,

$R^2$ für Wasserstoff, Alkyl oder gegebenenfalls substituiertes Phenyl steht,

$R^3$ für Wasserstoff, Alkyl, Alkenyl oder Alkinyl steht,

X für Halogen, Alkyl, Alkoxy, Alkylthio, Halogenalkyl, Halogenalkoxy oder Halogenalkylthio steht und

n für die Zahlen 0, 1 oder 2 steht,

dadurch gekennzeichnet, daß man Azolylethanole der Formel

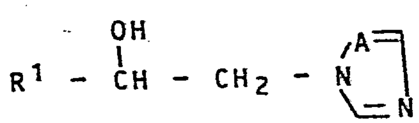

(II)

in welcher

A und $R^1$ die oben angegebene Bedeutung haben,

mit Benzylhalogeniden der Formel

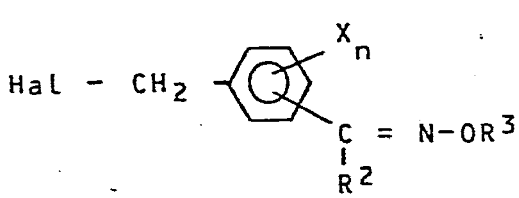

(III)

in welcher

Hal für Halogen, insbesondere Brom oder Chlor, steht und

$R^2$, $R^3$, X und n die oben angegebene Bedeutung haben,

in Gegenwart einer Base und in Gegenwart eines organischen Verdünnungsmittels oder in einem wässrig-organischen Zweiphasensystem in Gegenwart eines Phasentransferkatalysators umsetzt, und gegebenenfalls anschließend an die so erhaltenen Verbindungen der Formel (I) eine Säure oder ein Metallsalz addiert.

5. Fungizide Mittel, gekennzeichnet durch einen Gehalt an mindestens einem Azolylethyl-benzyl-ether-Derivat der Formel (I) in Ansprüchen 1 und 4.

6. Verfahren zur Bekämpfung von Pilzen, dadurch gekennzeichnet, daß man Azolylethyl-benzyl-ether-Derivate der Formel (I) in Ansprüchen 1 und 4 auf Pilze oder ihren Lebensraum einwirken läßt.

7. Verwendung von Azolylethyl-benzyl-ether-Derivaten der Formel (I) in Ansprüchen 1 und 4, zur Bekämpfung von Pilzen.

8. Verwendung von Azolylethyl-benzyl-ether-Derivaten der Formel (I) in Ansprüchen 1 und 4 als Pflanzenschutzmittel.

9. Verfahren zur Herstellung von fungiziden Mitteln, dadurch gekennzeichnet, daß man Azolylethyl-benzylether-Derivate der Formel (I) in Ansprüchen 1 und 4 mit Streckmitteln und/oder oberflächenaktiven Mitteln vermischt.